# EUROPEAN PATENT APPLICATION

(11) **EP 4 838 227 A1**
(43) Date of publication of application: **11.11.2026**
(21) Application number: 24915688.6
(22) Date of filing: 11.12.2024
(51) Int. Cl.: G01N 35/08

(54) **AUTOMATIC ANALYSIS DEVICE**

(30) Priority: 05.01.2024 JP 2024000740
(71) Applicant: Hitachi High-Tech Corporation, Minato-ku Tokyo 105-6409 (JP)
(72) Inventor: SAKANOUE, Kohei, Tokyo 105-6409 (JP); YAMASHITA, Nanae, Tokyo 105-6409 (JP); SUGIMURA, Yoshiaki, Tokyo 105-6409 (JP)
(74) Representative: MERH-IP Matias Erny Reichl Hoffmann Patentanwälte PartG mbB
(86) International application number: PCT/JP2024/043790
(87) International publication number: WO 2025/146765

(57) **Abstract**

The automatic analyzer includes: a flow channel (10) through which a sample containing a magnetic particle (13) bound to a specific substance is introduced into a capture region (14); supply means for supplying the sample to the flow channel (10); a magnet (19) that captures the magnetic particle (13); a slide mechanism (20) that moves the magnet (19) to a vicinity of a projection plane on which the capture region (14) is projected; a photodetector that detects the specific substance on the magnetic particle (13); discharge means for discharging the magnetic particle (13) from the flow channel (10) after the detection by the photodetector; and a magnet guide mechanism (24) that is formed from a magnetic material and moves the magnet (19).

## Description

### Technical Field

The present invention relates to an automatic analyzer.

### Background Art

An example of a method for detecting a target substance to be measured in an automatic analyzer is to immobilize the target substance to be measured onto a magnetic particle, bind a labeled substance to the target substance to be measured, and measure a signal derived from the label (see Patent Literature 1).

### Citation List

### Patent Literature

Patent Literature 1: International Unexamined Patent Application No. 2013/099648

### Summary of Invention

### Technical Problem

However, in the automatic analyzer described in Patent Literature 1, in capturing of a magnetic particle during measurement, no consideration is given to correction of the position of a magnet that affects the reproducibility of capturing a magnetic particle. The position where the magnetic particle is captured and the amount of the magnetic particle to be captured are affected by a capture operation position of the magnet. To reduce a variation in measurement and a variation in devices in analytical performance, it is necessary to position the magnet for capturing a magnetic particle at the same location relative to a detection region.

Therefore, an object of the present invention is to provide an automatic analyzer that reduces a variation in measurement and a variation in devices due to the position of the magnet, improves the reproducibility of capturing a magnetic particle in a detection region, and is capable of performing highly accurate measurement.

### Solution to Problem

According to the present invention, an automatic analyzer includes: a flow channel through which a sample containing a magnetic particle bound to a specific substance is introduced into a capture region; supply means for supplying the sample to the flow channel; a magnet that captures the magnetic particle; a slide mechanism that moves the magnet to a vicinity of a projection plane on which the capture region is projected; a detector that detects the specific substance on the magnetic particle; discharge means for discharging the magnetic particle from the flow channel after the detection by the detector; and a magnet guide mechanism that moves the magnet and is formed from a magnetic material.

### Advantageous Effects of Invention

According to the present invention, it is possible to provide an automatic analyzer that reduces a variation in measurement and a variation in devices due to the position of the magnet, improves the reproducibility of capturing a magnetic particle in a detection region, and is capable of performing highly accurate measurement.

### Brief Description of Drawings

[FIG. 1] FIG. 1 is a schematic configuration diagram of an immunoanalyzer.
[FIG. 2] FIG. 2 is a schematic configuration diagram of the immunoanalyzer using laser light.
[FIG. 3] FIG. 3 is a configuration diagram of the vicinity of the flow channel of the immunoanalyzer.
[FIG. 4A] FIG. 4A is a configuration diagram of the vicinity of the flow channel according to a first embodiment.
[FIG. 4B] FIG. 4B is a sectional view taken along the xy plane passing through a magnet guide mechanism according to the first embodiment.
[FIG. 4C] FIG. 4C is a sectional view taken along the xy plane passing through a magnet guide mechanism according to a modification of the first embodiment.
[FIG. 5A] FIG. 5A is a configuration diagram of the vicinity of the flow channel according to a second embodiment.
[FIG. 5B] FIG. 5B is a sectional view taken along the xy plane where a magnet and a channel wall are in contact with each other according to the second embodiment.
[FIG. 5C] FIG. 5C is a sectional view taken along the xy plane where a magnet and a channel wall are in contact with each other according to a modification of the second embodiment.
[FIG. 6A] FIG. 6A is a configuration diagram of the vicinity of the flow channel according to a third embodiment.
[FIG. 6B] FIG. 6B shows the positional relationship between a magnet guide mechanism and a magnet according to the third embodiment.
[FIG. 6C] FIG. 6C shows the positional relationship between a magnet guide mechanism and a magnet according to a modification of the third embodiment.
[FIG. 7A] FIG. 7A is a side view of a slide mechanism according to a fourth embodiment.
[FIG. 7B] FIG. 7B is a top view of the slide mechanism according to the fourth embodiment.
[FIG. 8] FIG. 8 is a configuration diagram of the vicinity of the flow channel according to a fifth embodiment.
[FIG. 9] FIG. 9 is a configuration diagram of the vicinity of the flow channel according to a sixth embodiment.

### Description of Embodiments

Embodiments of the present invention will be described below with reference to the drawings, but the present invention is not limited to the following embodiments and includes various modifications and applications within the technical scope of the present invention. As an example of an automatic analyzer, an immunoanalyzer will be described below. However, the present invention is not limited to an immunoassay, but is applicable to any automatic analyzer that captures a magnetic particle by switching magnetic field strength using the magnetic particle, and is a technology that is similarly applicable to analyzers for DNA, biochemistry, and the like.

### First Embodiment

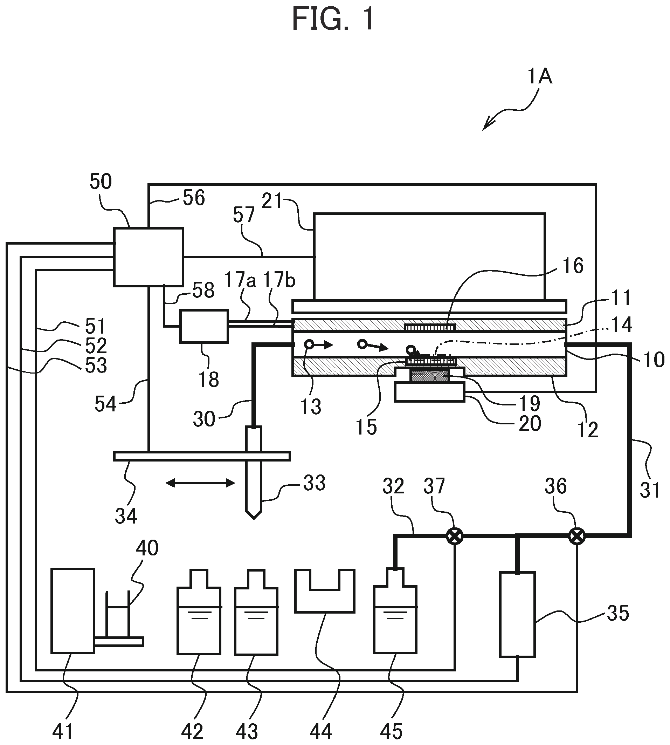

FIG. 1 is a schematic configuration diagram of an immunoanalyzer.

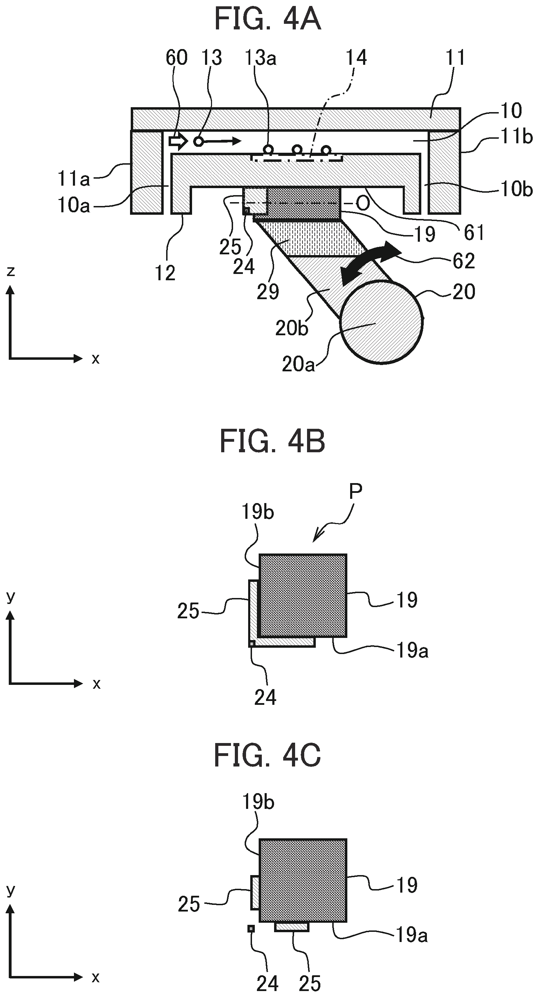

As shown in FIG. 1, the immunoanalyzer (automatic analyzer) 1A includes a flow channel 10 through which a sample containing a magnetic particle 13 bound to a specific substance is introduced into a capture region 14; a pump 35 that supplies the sample to the flow channel 10; a magnet 19 that captures the magnetic particle 13; a slide mechanism 20 that moves the magnet 19 to the vicinity of a projection plane 61 (see FIG. 4A) on which the capture region 14 is projected; a photodetector 21 (detector) that detects the specific substance on the magnetic particle 13; and the pump 35 that discharges the sample containing the magnetic particle 13 from the flow channel 10 after the detection by the photodetector 21. The pump 35 has both a function of supplying the sample to the flow channel 10 and a function of discharging the sample from the flow channel 10.

A sipper nozzle 33 is connected to the flow channel 10 via a tube 30. The pump 35 is connected to the flow channel 10 via a tube 31. An ON/OFF valve 36 is disposed in the tube 31 between the flow channel 10 and the pump 35. The pump 35 is controlled by a controller 50 via a signal line 52 and is capable of aspirating and discharging an accurate amount of liquid. The pump 35 is connected to a waste liquid container 45 via a tube 32. The tube 32 is connected to the tube 31 disposed between the valve 36 and the pump 35. An ON/OFF valve 37 is disposed in the tube 32. The valve 37 is controlled by the controller 50 via a signal line 51.

The sipper nozzle 33 is attached to an arm 34 so as to be movable by the arm 34. In addition, in a range in which the sipper nozzle 33 is moved, a suspension container 40, a cleaning liquid container 42, and a buffer solution container 43 are disposed. In addition, the suspension container 40 is disposed at a reaction unit 41. The reaction unit 41 is a unit for forming an immune complex composed of the magnetic particle 13 and a labeled substance, and has a structure for housing the suspension container 40. Further, a cleaning mechanism 44 has a function of cleaning the sipper nozzle 33.

The flow channel 10 includes a channel wall 11 at a top portion, a channel wall 12 at a bottom portion, the capture region 14 for the magnetic particle 13, a reaction field electrode 15, and a counter electrode 16. In addition, a member for increasing the height is sandwiched between the channel wall 11 and the channel wall 12. In the following description, the channel walls 11 and 12, the capture region 14, the reaction field electrode 15, and the counter electrode 16 may be collectively referred to as a flow cell. In other words, the flow cell is a structure (mechanism) that allows observation of a flowing object.

The channel wall 11 is formed from a transparent material. Each of the flow channels 11 and 12 may be formed, for example, using a planar plate, but are not limited thereto. The channel wall 11 is formed from a transparent material, allows light to pass through it, and allows observation of the internal flow state. The channel wall 11 may not be entirely transparent, and it suffices for only a portion through which light passes to be transparent as a window.

The channel wall 11 is preferably formed from a material that is substantially transparent to the wavelength of light emitted by the labeled substance of the magnetic particle complex (a mass of the object to be measured and the labeled substance) attracted to the capture region 14 in the flow cell, and is, for example, glass, plastic, or the like.

The capture region 14 where the magnetic particle 13 is captured is disposed in the flow channel 10. In the capture region 14, the reaction field electrode 15 is disposed. In addition, the counter electrode 16 is disposed on the opposite side of the reaction field electrode 15 (capture region 14) in the flow channel 10. The reaction field electrode 15 and the counter electrode 16 are connected voltage application means 18 via lead wires 17a and 17b. The voltage application means 18 are connected to the controller 50 via a signal line 58.

The magnet 19 is used as magnetic field application means in order to attract the magnetic particle 13 to the capture region 14. The magnet 19 is a permanent magnet or an electromagnet. When the magnetic particle 13 is to be attracted, for example, the magnet 19 is moved to a position immediately under the flow channel 10. For example, the magnet 19 is disposed on the slide mechanism 20 that can perform a circular motion, and the magnet 19 is moved to the position immediately under the flow channel 10 when the magnetic particle 13 is to be attracted.

The magnetic particle 13 is attracted to (captured in) the capture region 14 in the flow channel 10 due to a magnetic force exerted by the magnetic field of the magnet 19. In a case where the capture region 14 is a surface having a predetermined area, luminescence measurement is easily performed. In addition, the capture region 14 may be disposed on a bottom surface of the flow channel 10, another surface in the flow channel 10, or a plurality of surfaces three-dimensionally arranged.

When the inside of the flow channel 10 is to be cleaned, for example, the magnet 19 can be moved to a position where the effect of the magnet 19 in the flow channel 10 can be sufficiently reduced, whereby sufficient cleaning can be performed.

The controller 50 is connected to the valves 36 and 37, the pump 35, the arm 34, the voltage application means 18, the photodetector 21, and the slide mechanism 20 via a signal line 53, the signal line 51, the signal line 52, a signal line 54, the signal line 58, a signal line 57, and a signal line 56, respectively, and can independently control the valves 36 and 37, the pump 35, the arm 34, the voltage application means 18, the photodetector 21, the slide mechanism 20.

In measurement, the voltage application means 18 is controlled by the controller 50. Accordingly, a voltage is applied between the reaction field electrode 15 and the counter electrode 16 in the flow channel 10. This enables the labeled substance bound to the magnetic particle 13 attracted to the reaction field electrode 15 (capture region 14) to electrochemically emit light. As long as the channel wall 11 is formed from a transparent material around the counter electrode 16 such that measurement by the photodetector 21 is possible, other portions do not need to be formed from a transparent material.

Each of the reaction field electrode 15 and the counter electrode 16 can be formed from a material such as gold, platinum, palladium, tungsten, iridium, or nickel, an alloy thereof, or a carbon material. In addition, the reaction field electrode 15 and the counter electrode 16 may be formed from a material obtained by depositing the above-described material by plating, spattering, or the like on a base material such as titanium.

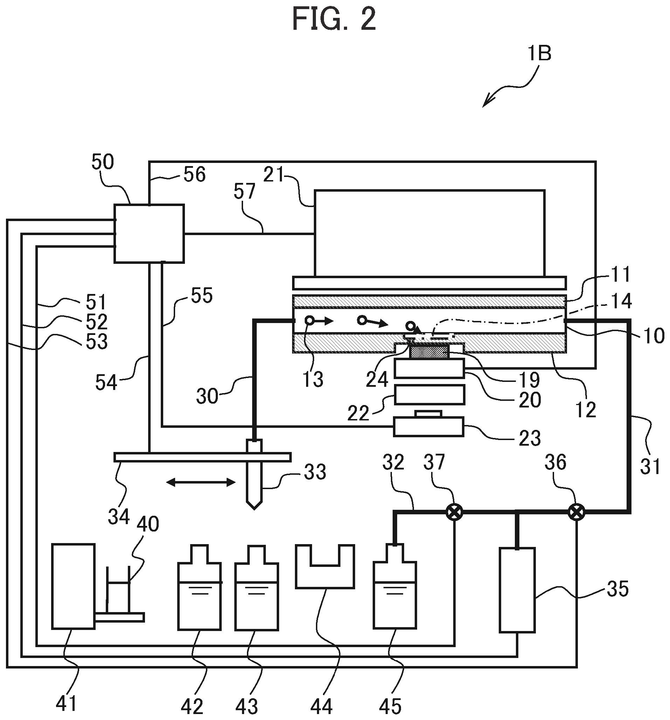

By employing a luminescence method using the counter electrode 16, the reaction field electrode 15 and the counter electrode 16 can be fixed within the flow cell. Compared with an immunoanalyzer 1B using laser light as described below with reference to FIG. 2, the immunoanalyzer 1A shown in FIG. 1A does not require a condenser lens 22 and a laser light source 23, eliminates the need for laser light positioning and the selection and installation of a transparent material for the capture region 14, and thus it can be expected to simplify the device and suppress a variation in light emission.

For example, a camera or a photomultiplier tube can be used for the photodetector 21.

The sample to be analyzed is a biological substance such as serum or urine. In a case where the sample is serum, a specific component to be analyzed may be, for example, a tumor marker, an antibody, an antigen-antibody complex, or a single protein. In the following description, it is assumed that the specific component is thyroid stimulating hormone (TSH).

The suspension container 40 contains a suspension obtained by mixing the sample to be analyzed with a bead solution and a reagent as a pretreatment step, and then causing the mixture to react at a constant temperature (for example, 37 degrees) for a certain period of time. The bead solution is a solution in which the magnetic particle 13 made by embedding a particulate magnetic material in a matrix material such as polystyrene is dispersed in a buffer solution. Streptavidin, which can bind to biotin, is bound to the surface of the matrix material. The reagent contains a substance that binds the magnetic particle 13 to the specific component TSH in the sample, and contains an anti-TSH antibody with a biotinylated end. The reagent differs depending on the type of the specific component to be analyzed, and for example, an immunoglobulin, an antigen, an antibody, or another biological substance is used as the reagent.

In the cleaning liquid container 42, a cleaning liquid for cleaning the inside of the flow channel 10 and the inside of the tube 30 is stored.

Although the immunoanalyzer 1A (see FIG. 1) is described above, the immunoanalyzer that uses an excitation light source will be described with reference to FIG. 2 as an example of a device according to a modification in which a mechanism is different. FIG. 2 is a schematic configuration diagram of the immunoanalyzer that uses laser light as an example of excitation light.

As shown in FIG. 2, the immunoanalyzer 1B includes the condenser lens 22 and the laser light source 23 instead of the reaction field electrode 15, the counter electrode 16, and the voltage application means 18 disposed in the immunoanalyzer 1A. The laser light source 23 is connected to the controller 50 via a signal line 55. The same configurations in the immunoanalyzer 1B as those in the immunoanalyzer 1A shown in FIG. 1 are denoted by the same reference signs as those used for the immunoanalyzer 1A, and a duplicate description will be omitted.

The laser light source 23 and the condenser lens 22 are disposed around the bottom portion of the flow channel 10. The channel walls 11 and 12 of the flow cell are formed from a transparent material. On the capture region 14 disposed in the channel walls 11 and 12 of the flow cell, laser light radiated from the bottom portion of the flow channel 10 via the laser light source 23 and the condenser lens 22 is condensed. In this case, the capture region 14 is preferably formed from a material that is substantially transparent to the wavelength of light emitted by the labeled substance of the magnetic particle complex attracted to the capture region 14 within the flow cell such that laser light can be radiated onto the capture region 14. For example, the capture region 14 is preferably formed from glass, plastic, or the like.

In addition, for example, in a case where the laser light source 23 and the condenser lens 22 are configured such that laser light does not need to pass through the capture region 14 due to an idea for arrangement or the like, the capture region 14 is preferably formed from a material, such as gold, platinum, or carbon, with excellent mechanical strength, corrosion resistance, and processing efficiency in consideration of the attraction of the magnetic particle complex onto its upper surface. In addition, in a case where laser light does not need to pass through the capture region 14, the channel wall 12 may not be formed from a transparent material, and may be formed from a material such as ceramics, metal, or plastic.

When emitted light is to be measured, the magnetic force within the flow channel 10 is released by stopping a fluid in the flow channel 10 and retracting the magnet 19 from the position immediately under the flow channel 10 by the slide mechanism 20. Since the flow is stopped even when the magnetic force is released, the magnetic particle 13 is held in the capture region 14. Therefore, while the magnetic particle 13 is held in the capture region 14, luminescence by irradiation with laser light can be induced. By receiving light emitted from the labeled substance bound to the magnetic particle 13 with the photodetector 21, the measurement can be performed.

The labeled substance in the immunoanalyzer 1B shown in FIG. 2 may be different from the labeled substance in FIG. 1. A specific example of the labeled substance can be appropriately designed by those skilled in the art based on a known technique.

Further, an operation of the automatic analyzer is described using the immunoanalyzer shown in FIG. 1 as an example. One cycle of an analysis includes a suspension aspiration period, a magnetic particle attraction period, a detection period, a cleaning period, a reset period, and a preliminary aspiration period. The one cycle is started when the suspension container 40 storing the suspension treated in the reaction unit 41 is set at a predetermined position.

In the suspension aspiration period, a signal from the controller 50 opens the valve 36 and sets the valve 37 to its closed state. Then, a signal from the controller 50 causes the arm 34 to operate so as to insert the sipper nozzle 33 into the suspension container 40. Subsequently, a signal from the controller 50 causes the pump 35 (supply means) to perform an operation of aspirating a constant amount. Therefore, the suspension within the suspension container 40 is introduced into the tube 30 through the sipper nozzle 33. In this state, the pump 35 is stopped and the arm 34 is operated to insert the sipper nozzle 33 into the cleaning mechanism 44. The sipper nozzle 33 is cleaned while passing through the cleaning mechanism 44.

In the magnetic particle attraction period, a signal from the controller 50 causes the slide mechanism 20 to operate to move the magnet 19 to the bottom portion of the flow channel 10. Then, a signal from the controller 50 causes the pump 35 to perform aspiration at a constant speed. During the period, the suspension present in the tube 30 passes through the flow channel 10. Since the magnetic field from the magnet 19 occurs in the channel walls 11 and 12, the magnetic particle 13 contained in the suspension is aspirated toward the magnet 19 due to the magnetic force and captured in the capture region 14. After a fixed period of time elapses, the aspiration of the suspension by the pump 35 is stopped. That is, the flow in the flow channel 10 (flow cell) is stopped.

In the detection period, a signal from the controller 50 causes the slide mechanism 20 to operate to move the magnet 19 away from the flow channel 10. Then, a signal from the controller 50 controls the voltage application means 18 so as to apply a voltage between the reaction field electrode 15 and the counter electrode 16 within the flow channel 10. This enables the labeled substance bound to the magnetic particle 13 attracted on the reaction field electrode 15 (capture region 14) to electrochemically emit light. The emitted light is optionally wavelength-selected by a filter and detected by the photodetector 21 such as a camera or a photomultiplier tube. The intensity of the detected emitted light is collected by the controller 50 as a signal. After a fixed period of time elapses, the application of the voltage is stopped. In the detection period, the arm 34 is operated and the sipper nozzle 33 is inserted into the cleaning mechanism 44.

In the cleaning period, a signal from the controller 50 causes the pump 35 to perform aspiration so as to cause the cleaning liquid aspirated from the cleaning liquid container 42 to pass through the flow channel 10. In this case, the magnet 19 is away from the flow channel 10 and thus the magnetic particle 13 is not held in the capture region 14 and is washed away together with the cleaning liquid.

In the reset period, a signal from the controller 50 closes the valve 36, opens the valve 37, and causes the pump 35 (discharge means) to perform a discharge operation. Therefore, the liquid within the pump 35 is discharged into the waste liquid container 45.

In the preliminary aspiration period, a signal from the controller 50 causes the buffer solution to be aspirated from the buffer solution container 43 so as to fill the buffer solution into the tube 30 and the flow channel 10. After the preliminary aspiration period, the next one cycle can be started.

As described above, the immunoanalyzer (automatic analyzer) 1A includes the flow channel 10 through which a sample liquid (sample) containing the magnetic particle 13 bound to the specific substance (substance to be analyzed) is introduced into the capture region 14; the pump 35 (supply means) that supplies the sample liquid to the flow channel 10; the magnet 19 (capture means) that has a magnetic field structure for generating a magnetic field and attracts the magnetic particle 13 to the capture region 14 by the magnetic field; the photodetector 21 (detector) that measures the specific substance attracted to the capture region 14; and the pump 35 (discharge means) that discharges the magnetic particle 13 from the flow channel 10 after the measurement by the photodetector 21. Note that both the supply means and the discharge means are not limited to the pump 35 and individual components may be used for the supply means and the discharge means. In this case, the sample liquid is a liquid containing the substance to be analyzed and is, for example, the suspension.

Instead of the configuration described above, a portion of a known automatic analyzer may be used.

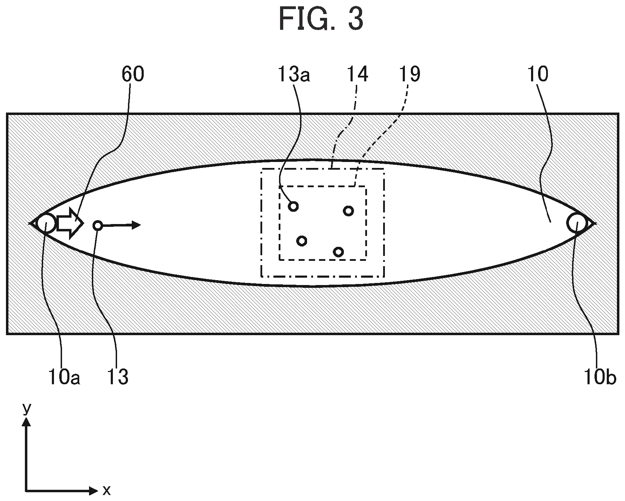

Regarding the immunoanalyzer 1A described above, a flow cell structure and a structure around the magnet will be described with reference to FIGS. 3 and 4. FIG. 3 is a configuration diagram of the vicinity of the flow channel of the immunoanalyzer. FIG. 4A is a configuration diagram of the vicinity of the flow channel according to the first embodiment. FIG. 4B is a sectional view taken along an xy plane passing through the magnet guide mechanism according to the first embodiment. FIG. 4C is a sectional view taken along the xy plane passing through a magnet guide mechanism according to a modification of the first embodiment. Note that the direction of the flow within a central portion of the flow cell is defined as an x axis, a horizontal direction of a cross section perpendicular to the flow is a y axis, and a vertical direction of the same cross section is a z axis. FIG. 4A does not illustrate the reaction field electrode 15 and the counter electrode 16 and illustrates only the capture region 14 (the same applies to the second embodiment and the subsequent embodiments).

As shown in FIG. 3, the flow channel 10 is configured such that the width of the flow channel in the xy plane gradually increases from an end where the suspension is introduced toward a central portion, and gradually decreases from the central portion to an end where the suspension is discharged. In addition, the flow channel 10 is configured such that the magnet 19 is placed at the central portion of the flow channel 10 in the x direction by the slide mechanism 20. The capture region 14 where the magnetic particle 13 is captured has an area larger than that of the magnet 19, for example.

As shown in FIG. 4A, the suspension containing the magnetic particle 13 flows in a +z direction (upward direction in the drawing) along a passage 10a formed by a channel wall 11a and the channel wall 12 and enters the flow cell. Then, the suspension flows in a +x direction as indicated by a direction 60 (see a white arrow) of the flow within the flow channel 10 and reaches the end of the flow channel 10. Then, the suspension flows in a -z direction (downward direction in the drawing) along a passage 10b formed by a channel wall 11b and the channel wall 12 and is discharged from the flow cell. During this period of time, the suspension receives the magnetic field of the magnet 19, and the magnetic particle 13 is captured on the surface of the capture region 14 due to the magnetic force. In FIG. 4A, the captured magnetic particle is denoted by reference sign 13a.

The immunoanalyzer 1A according to the first embodiment includes the magnet guide mechanism 24 disposed near the flow channel 10 and formed from a magnetic material, a stopper structure 25 that stops the movement of the magnet 19, and the slide mechanism 20 that has a deformable portion 29 that is deformable due to a magnetic force received by the magnet 19.

The slide mechanism 20 has a mechanism that moves the magnet 19 in a direction in which the magnet 19 is moved away from the channel wall 12. An arm 20b is formed on a rotation shaft 20a, and the magnet 19 is disposed via the deformable portion 29. Although not shown, a signal from the controller 50 enables the rotation shaft 20a to move in a direction indicated by an arrow 62. The magnet 19 is disposed at a distal end of the arm 20b. When the rotation shaft 20a rotates counterclockwise, the magnet 19 moves away from the channel wall 12. When the rotation shaft 20a rotates clockwise, the magnet 19 approaches the channel wall 12.

In the structure shown in FIG. 4A, in the magnetic particle attraction period, when the slide mechanism 20 operates and the magnet 19 moves to the bottom portion of the flow channel 10, the magnet 19 receives the magnetic force from the magnet guide mechanism 24. In this case, the deformable portion 29 included in the slide mechanism 20 deforms and the magnet 19 moves toward the magnet guide mechanism 24. The moved magnet 19 contacts the stopper structure 25 and stops, and the position of the magnet 19 is corrected.

Since a general magnetic material can be used as the material of the magnet guide mechanism 24, the material of the magnet guide mechanism 24 may be a hard magnetic material, a soft magnetic material, or a combination thereof. In a case where the hard magnetic material is used, the magnet 19 may be moved by either an attractive force or a repulsive force. However, the soft magnetic material is preferably used from the viewpoint of reducing the effect of the magnet guide mechanism 24 on the capturing of the magnetic particle. The soft magnetic material is soft iron or the like.

In a case where the magnet guide mechanism 24 is present on the channel wall 12 (on the outer periphery side), the magnet guide mechanism 24 may adhere to and be fixed to the channel wall 12 with an adhesive such as a curable resin, or may be fixed by having a structure in which the channel wall 12 holds the magnet guide mechanism 24. In addition, in a case where the magnet guide mechanism 24 is present inside the channel wall 12, the magnet guide mechanism 24 may be fixed inside the channel wall 12 by insert molding or the like.

The size of the magnet guide mechanism 24 is not limited as long as the magnet guide mechanism 24 moves the magnet 19 by the magnetic force. However, from the viewpoint of reducing consumption of a magnetic flux of the magnet 19 used to capture the magnetic particle by the magnet guide mechanism 24, it is preferable that the magnet guide mechanism 24 be made as small as possible.

In addition, the magnet guide mechanism 24 may be an additional member having a simple structure such as a cube or a sphere to the flow cell or a peripheral member of the flow cell or may be a structure obtained by machining. Further, from the viewpoint of reducing consumption of the magnetic flux of the magnet 19 used to capture the magnetic particle by the magnet guide mechanism 24, it is preferable that the position of the magnet 19 be corrected at as few points or locations as possible.

As shown in FIG. 4B, the stopper structure 25 is formed in a substantially L shape and configured to contact a surface 19a of the magnet 19 facing in the y-axis direction and a surface 19b of the magnet 19 facing in the x-axis direction. In this manner, the stopper structure 25 simultaneously contacts the two surfaces of the magnet 19 facing in the x-axis direction and the y-axis direction (at two or more locations).

The shape of the stopper structure 25 may be a rectangular parallelepiped, a cylindrical shape, or any shape that allows contact with the magnet 19 at two or more points at the same time as long as the stopper structure 25 stops the magnet 19 moved by the magnet guide mechanism 24.

In addition, the stopper structure 25 does not necessarily have to be a continuous structure (see FIG. 4B) but stopper structures 25 present at two or more points may contact the magnet 19 at two or more locations, as shown in FIG. 4C. However, from the viewpoint of correcting the position of the magnet 19, in a case where the magnet 19 is a polyhedron, it is preferable that the stopper structure 25 contact the magnet 19 at a point included in two different surfaces, a line, or a surface. Further, in a case where the magnet 19 is cylindrical and the stopper structure 25 contacts a curved surface, it is preferable that the stopper structure 25 contact the magnet 19 at a position where the movement of the magnet 19 in the x-axis direction and the y-axis direction can be stopped.

The magnet guide mechanism 24 is not limited to be present in or on the channel wall 12, and may be fixed to the outside without contacting other flow channel components such as the channel wall 11 or the flow cell, as long as the magnet guide mechanism 24 moves the magnet 19 by the magnetic force. However, regarding the position in the z-axis direction, from the viewpoint of reducing the consumption of the magnetic flux of the magnet 19 used to capture the magnetic particle by the magnet guide mechanism 24, it is preferable that the magnet guide mechanism 24 be positioned in a -z direction relative to the center O of the magnet 19 in the z-axis direction when the magnet 19 is in contact with the projection plane 61 on which the capture region 14 is projected. Note that the -z direction is a direction away from the projection plane 61. In a case where the magnet guide mechanism 24 is formed from a single additional member, for positioning of the magnet guide mechanism 24 in the x-axis direction and the y-axis direction, it is preferable that the magnet guide mechanism 24 be positioned such that, when the magnetic force acts between the magnet guide mechanism 24 and the magnet 19, the magnet 19 moves in a direction where the magnet 19 contacts the stopper structure 25 at two or more points due to the magnetic force.

The stopper structure 25 may be formed from any material that does not deform when the stopper structure 25 comes into contact with the magnet 19. The stopper structure 25 may be formed from the same material as the channel wall 12 or may be formed from a different material, such as ceramics, metal, or plastic, from that of the channel wall 12. The stopper structure 25 may be a separate member fixed to the channel wall 12 or may be a portion of the structure of the channel wall 12.

The motion form of the slide mechanism 20 is not limited and may use forms such as a linear motion in the horizontal or the vertical direction or a circular motion. However, it is preferable that the magnet 19 be movable in all the x-, y-, and z-axis directions through the movement of the slide mechanism 20 near the projection plane 61 on which the capture region 14 is projected, and the movement of the magnet 19 by the magnet guide mechanism 24.

It suffices for the deformable portion 29 of the slide mechanism 20 to deform when the magnet 19 receives the magnetic force from the magnet guide mechanism 24. For example, a material for forming the deformable portion 29 may have a rod-shaped structure formed from a general elastic material such as elastomer, rubber, or resin, or the slide mechanism 20 may be formed from elastomer, rubber, resin, or metal and have a spring-like structure. The deformable portion 29 formed from an elastic material may be a portion of the slide mechanism 20 (see FIG. 4A) or the entire slide mechanism 20.

The shape of the magnet 19 is not limited and may be a rectangular parallelepiped (see FIG. 4A) or a sphere. The magnetization direction of the magnet 19 is not limited and may be parallel to the z axis or perpendicular to the z axis, for example.

The immunoanalyzer 1A according to the first embodiment described above includes the flow channel 10 through which the sample containing the magnetic particle 13 bound to the specific substance (substance to be analyzed) is introduced into the capture region 14, the pump 35 (supply means) that supplies the sample to the flow channel 10, the magnet 19 that captures the magnetic particle 13, the slide mechanism 20 that moves the magnet 19 to the vicinity of the projection plane 61 on which the capture region 14 is projected, the photodetector 21 (detector) that detects the specific substance on the magnetic particle 13, the discharge means for discharging the magnetic particle 13 from the flow channel 10 after the detection by the photodetector 21, and the magnet guide mechanism 24 that moves the magnet 19 and is formed from a magnetic material (see FIGS. 1 and 4A). Accordingly, a variation in measurement and a variation in devices due to the position of magnet 19 are reduced, the reproducibility of capturing the magnetic particle 13 within the detection region (capture region 14) is improved, and the immunoanalyzer 1A capable of high-precision measurement can be provided. Furthermore, in the immunoanalyzer 1B shown in FIG. 2 that uses excitation light, the irradiation range of the excitation light can be narrowed by improving the reproducibility of capturing the magnetic particle 13, and the laser output can be reduced.

In the first embodiment, the magnetic material is a soft magnetic material. Accordingly, it is possible to reduce the effect on the capture of the magnetic particle by the magnet guide mechanism 24.

In the first embodiment, the deformable portion 29 that is at least a portion of the slide mechanism 20 is formed from an elastic material (see FIG. 4A). Accordingly, the deformation of the deformable portion 29 can correct the position of the magnet 19.

In the first embodiment, the magnet guide mechanism 24 is positioned below the center O of the magnet 19 when the magnet 19 is positioned at a capture operation position P (see FIGS. 4A and 4B). Accordingly, the magnet guide mechanism 24 can reduce the consumption of the magnetic flux of the magnet 19 used to capture the magnetic particle.

In the first embodiment, the stopper structure 25 for stopping the magnet 19 moved by the magnet guide mechanism 24 is disposed (see FIG. 4B). Accordingly, the magnet 19 can be corrected at the same location and the reproducibility of capturing the magnetic particle 13 can be improved.

In the modification of the first embodiment, the stopper structure 25 has a shape that simultaneously contacts the magnet 19 at at least two or more points (see FIG. 4C). Accordingly, compared to a case where the magnet 19 is corrected at one point, the magnet 19 is easily corrected at the same location (the position is easily corrected).

### Second Embodiment

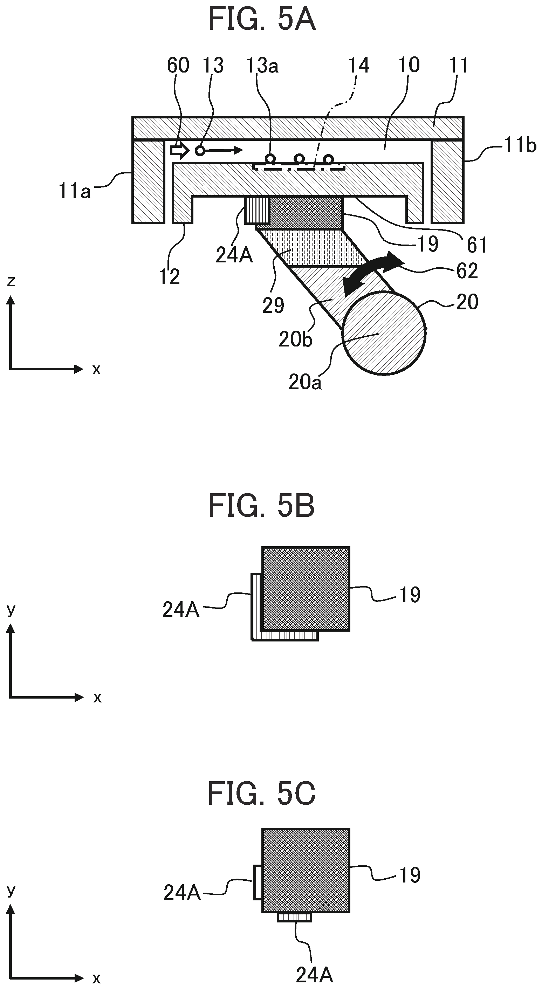

FIG. 5A is a configuration diagram of the vicinity of a flow channel according to a second embodiment. FIG. 5B is a sectional view taken along the xy plane where a magnet and a channel wall are in contact with each other according to the second embodiment. FIG. 5C is a sectional view taken along the xy plane where a magnet and a channel wall are in contact with each other according to a modification of the second embodiment.

In a structure shown in FIG. 5A, in the magnetic particle attraction period, when a slide mechanism 20 operates and the magnet 19 is moved to a bottom portion of the flow channel 10, the magnet 19 receives a magnetic force from a magnetic guide mechanism 24A. In this case, a deformable portion 29 included in the slide mechanism 20 deforms and the magnet 19 is moved. The moved magnet 19 contacts the magnet guide mechanism 24A and stops, and the position of the magnet 19 is corrected. In the second embodiment, a flow cell does not include the stopper structure 25 described in the first embodiment, and the magnet guide mechanism 24A has a configuration for stopping the movement of the magnet 19 and correcting the position of the magnet 19.

As shown in FIG. 5B, the magnet guide mechanism 24A is formed in a substantially L-shape and from a magnetic material and protrudes from the channel wall 12 in the vicinity of a projection plane 61 on which a capture region 14 is projected. In the second embodiment, in a case where a hard magnetic material is used as the magnetic material, it is necessary to use an attractive force to move the magnet 19.

The shape of the magnet guide mechanism 24A may be a rectangular parallelepiped, a cylindrical shape, or any shape that allows contact with the magnet 19 at two or more points at the same time as long as the magnet guide mechanism 24A stops the magnet 19 to be moved. The magnet guide mechanism 24A may not have a continuous structure, and may be present at two or more positions and may contact the magnet 19 at two or more points as shown in FIG. 5C. However, from the viewpoint of correcting the position of the magnet 19, in a case where the magnet 19 is a polyhedron, it is preferable that the magnet guide mechanism 24A contact the magnet 19 at a point included in two different surfaces, a line, or a surface. Further, in a case where the magnet 19 is cylindrical and the magnet guide mechanism 24A contacts a curved surface, it is preferable that the magnet guide mechanism 24A contact the magnet 19 at a position where the movement of the magnet 19 in the x-axis direction and the y-axis direction can be stopped.

In the second embodiment described above, it is possible to obtain the same effects as those in the first embodiment. In the second embodiment, the magnet guide mechanism 24A and the magnet 19 simultaneously contact each other at at least two or more points (see FIG. 5C). Accordingly, the configuration in which the stopper structure 25 is omitted can be obtained.

### Third Embodiment

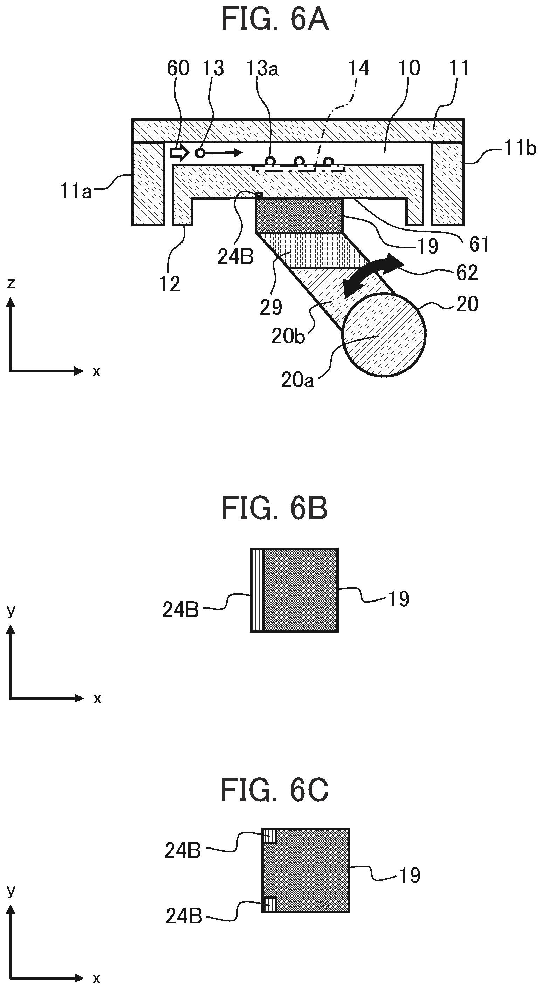

FIG. 6A is a configuration diagram of the vicinity of a flow channel according to a third embodiment. FIG. 6B is a diagram showing the positional relationship between a magnet guide mechanism and a magnet according to the third embodiment. FIG. 6C is a diagram showing the positional relationship between a magnet guide mechanism and a magnet according to a modification of the third embodiment.

In a structure shown in FIG. 6A, in the magnetic particle attraction period, when a slide mechanism 20 operates and the magnet 19 moves to a bottom portion of the flow channel 10, the magnet 19 receives a magnetic force from a magnetic guide mechanism 24B. In this case, a deformable portion 29 included in the slide mechanism 20 deforms and the magnet 19 is moved. The moved magnet 19 is attracted to the magnet guide mechanism 24B and stops, and the position of the magnet 19 is corrected.

The magnet guide mechanism 24B is disposed in a channel wall 12. In this case, the magnet guide mechanism 24B is fixed inside the channel wall 12 (within the wall) by insert molding or the like.

As shown in FIG. 6B, the magnet guide mechanism 24B is formed from a magnetic material and is positioned between a reaction field electrode 15 and a projection plane 61 on which a capture region 14 is projected. In the third embodiment, in a case where a hard magnetic material is used as the magnetic material, it is necessary to use an attractive force to move the magnet 19. In order to ensure the strength of the magnetic force acting between the magnet guide mechanism 24B and the magnet 19 while a magnetic field for capturing a magnetic particle 13 is not weakened, it is preferable that the magnet guide mechanism 24B be positioned inside the channel wall 12 and be exposed from the channel wall 12 so as to match the projection plane 61 on which the capture region 14 is projected.

The number of points where the magnet guide mechanism 24B contacts the magnet 19 is not limited. The magnet guide mechanism 24B may attract the magnet 19 at one point as shown in FIG. 6B, or the magnet guide mechanism 24B that is not continuous may attract the magnet 19 at two or more points as shown in FIG. 6C. From the viewpoint of correcting the position of the magnet 19, as shown in FIG. 6B, in a case where the magnet guide mechanism 24B attracts the magnet 19 at one point, it is preferable that a dimension of a member of the magnet guide mechanism 24B match a dimension of the magnet 19 in one or more parts. As shown in FIG. 6C, in a case where the magnet guide mechanism 24B attracts the magnet 19 at two or more points, it is preferable that a dimension of a member of the magnet guide mechanism 24B or a dimension of a gap between members of the magnet guide mechanism 24B match a dimension of the magnet 19 in one or more parts.

The magnet 19 does not necessarily need to be stopped by the balance between the resistance and the magnetic force when the magnet 19 comes into contact with the magnet guide mechanism 24B or the stopper structure 25. As shown in FIG. 6C, the magnet 19 may be stopped by balancing magnetic forces between the magnet guide mechanism 24B and the magnet 19 at a plurality of points.

In the third embodiment described above, it is possible to obtain the same effects as those in the second embodiment. In the third embodiment, the magnet guide mechanism 24B is fixed to the channel wall 12 (inside the wall) of the flow channel 10 by insert molding (see FIG. 6A). Accordingly, it is possible to suppress a variation in the position of the magnet guide mechanism 24B as compared to a case where the magnet guide mechanism 24B is attached to the channel wall 12 by another member.

In the third embodiment, the magnet guide mechanism 24B shares the shape with at least one side of the magnet 19 on a projection plane 61 in at least one or more directions (see FIG. 6B). Accordingly, it is possible to correct the position of the magnet 19.

In the third embodiment, the magnet guide mechanism 24B is a member fixed near the flow channel 10 and is present at two positions (see FIG. 6C). The magnet guide mechanism 24B may be present at three or more positions. Accordingly, it is possible to correct the position of the magnet 19.

### Fourth Embodiment

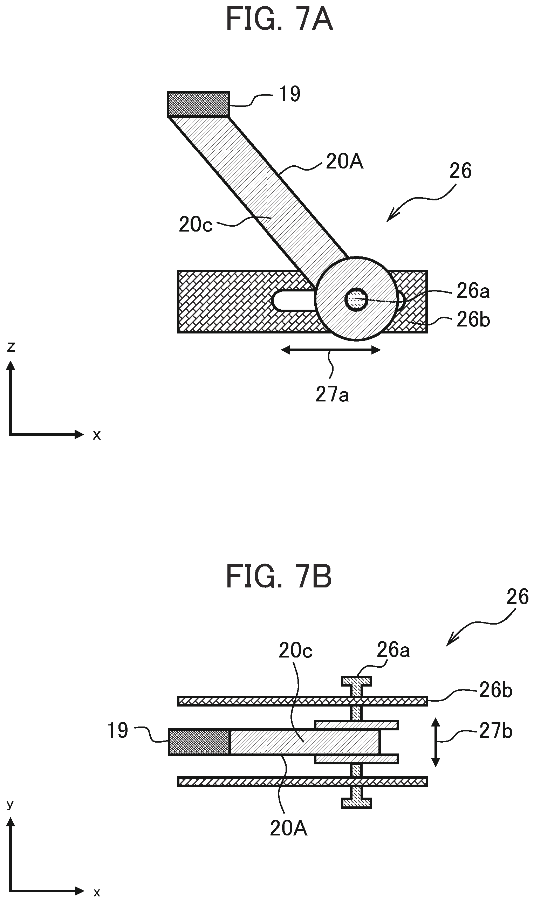

FIG. 7A is a side view of a slide mechanism according to a fourth embodiment. FIG. 7B is a top view of the slide mechanism according to the fourth embodiment.

As shown in FIGS. 7A and 7B, the slide mechanism 20A according to the fourth embodiment includes a movable structure 26 that is in a drive unit and is movable in the x-axis direction and the y-axis direction. In the movable structure 26, a magnet 19 is fixed to a distal end of an arm 20c and a shaft 26a is disposed at a base end of the arm 20c. A guide member 26b that slidably guides the shaft in the direction (x-axis direction) indicated by an arrow 27a is disposed at both sides of the arm 20c. The arm 20c is configured to move in the direction (y-axis direction) indicated by an arrow 27b. Note that, in the slide mechanism 20A according to the fourth embodiment, the deformable portion 29 described in the first embodiment is not disposed.

In a structure shown in FIGS. 7A and 7B, in the magnetic particle attraction period, when the slide mechanism 20A operates and the magnet 19 moves to a bottom portion of a flow channel 10, the magnet 19 receives a magnetic force from a magnet guide mechanism 24. In this case, the slide mechanism 20A in the movable structure 26 moves in the x-axis direction or the y-axis direction to move the magnet 19.

The movable structure 26 may not be the structure shown in FIGS. 7A and 7B. The movable structure 26 may be a structure that allows the slide mechanism 20A to move in the x-, y-, or z-axis direction by complementing the direction in which the movement by the slide mechanism 20A does not enable the magnet 19 to move.

In the fourth embodiment described above, it is possible to obtain the same effects as those in the first embodiment. In the fourth embodiment, a portion of the slide mechanism 20A includes the movable structure 26 that allows the slide mechanism 20A to move in the x-axis direction and the y-axis direction (at least one or more axis directions). Accordingly, it is possible to correct the position of the magnet 19.

### Fifth Embodiment

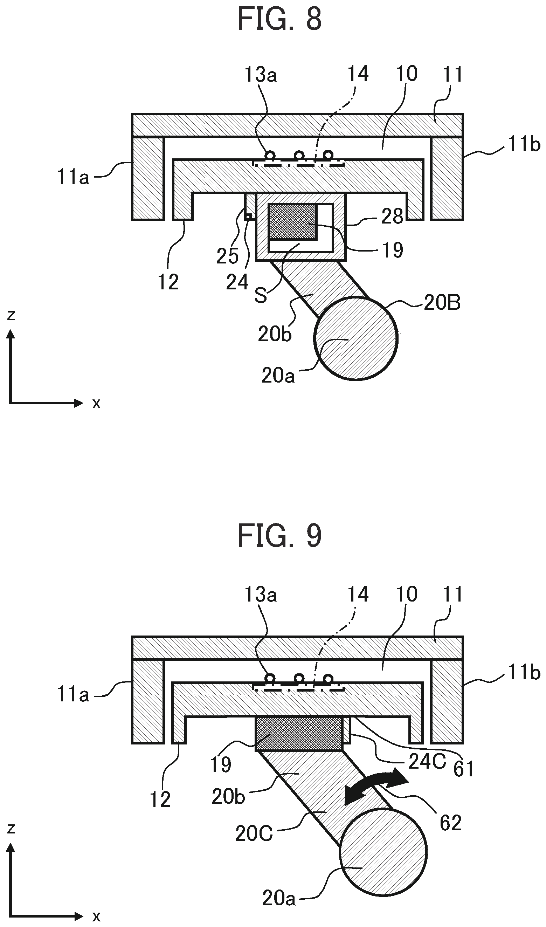

FIG. 8 is a configuration diagram of the vicinity of a flow channel according to a fifth embodiment.

As shown in FIG. 8, a slide mechanism 20B used in the fifth embodiment includes an outer shell 28 enclosing a magnet 19. The outer shell 28 has a gap S between the outer shell 28 and the magnet 19. By providing the gap S, the magnet 19 is configured to be movable within the outer shell 28 in the x-axis direction and the z-axis direction. That is, in the fifth embodiment, the magnet 19 is not fixed to the slide mechanism 20B.

In a structure shown in FIG. 8, in the magnetic particle attraction period, when the slide mechanism 20B operates and the magnet 19 moves to a bottom portion of a flow channel 10, the magnet 19 receives a magnetic force from a magnet guide mechanism 24, and in a space within the outer shell 28, only the magnet 19 moves. In the detection period, in order for the slide mechanism 20B to operate to move the magnet 19 away from the flow channel 10, the magnet 19 is not continuously attracted to the magnet guide mechanism 24 and is pulled away from the magnet guide mechanism 24 due to the outer shell 28.

The outer shell 28 may completely enclose the magnet 19 or may be shaped such that a portion of the magnet 19 is exposed if the magnet 19 does not detach from inside the outer shell 28.

In the fifth embodiment described above, it is possible to obtain the same effects as those in the first embodiment. In the fifth embodiment, the slide mechanism 20B includes the outer shell 28 that encloses at least a portion of the magnet 19, the gap S is formed between the magnet 19 and the outer shell 28, and the magnet 19 moves within the outer shell 28. Accordingly, it is possible to correct the position of the magnet 19.

### Sixth Embodiment

FIG. 9 is a configuration diagram of the vicinity of a flow channel according to a sixth embodiment.

As shown in FIG. 9, in the sixth embodiment, a magnet guide mechanism 24C is a member fixed on a channel wall 12.

In a structure shown in FIG. 9, a slide mechanism 20C operates as indicated by an arrow 62 in the magnetic particle attraction period. When the magnet 19 approaches a bottom portion of the flow channel 10, the magnet 19 is pressed against the magnet guide mechanism 24, and the operation of the magnet 19 and the slide mechanism 20C stops.

The magnet guide mechanism 24C is formed from a material that is not a magnetic material and does not deform when the magnet guide mechanism 24C stops the magnet 19. The magnet guide mechanism 24C may be formed from the same material as the channel wall 12 or may be formed from a different material, such as ceramics, metal, or plastic, from that of the channel wall 12. A constraint and a preferred form for the shape of the magnet guide mechanism 24 are the same as those in the second embodiment.

In the sixth embodiment, the example in which the movement of the slide mechanism 20C indicated by the arrow 62 is circular movement is described, but the movement of the slide mechanism 20C may be along with horizontal movement or vertical movement.

In the sixth embodiment described above, the flow channel 10 through which the sample containing the magnetic particle 13 bound to the specific substance is introduced into a capture region 14, a pump 35 (supply means) that supplies the sample to the flow channel 10, the magnet 19 that captures the magnetic particle 13, the slide mechanism 20C that moves the magnet 19 to the vicinity of a projection plane 61 on which the capture region 14 is projected, a photodetector 21 that detects the specific substance on the magnetic particle 13a captured on a surface of the capture region 14, and the pump 35 (discharge means) that discharges the magnetic particle 13 from the flow channel 10 after the detection by the photodetector 21 are disposed. The magnet 19 moved by driving of the slide mechanism 20C is pressed against the magnet guide mechanism 24C present in the vicinity of the projection plane 61 on which the capture region 14 is projected, and is stopped (see FIG. 9). In the embodiment different from the first embodiment as described above, it is possible to provide an immunoanalyzer 1A in which a variation in measurement and a variation in devices due to the position of the magnet 19 are reduced without consumption of a magnetic flux of the magnet 19 used to capture the magnetic particle, the reproducibility of capturing the magnetic particle 13 in the detection region is improved, and highly accurate measurement can be performed.

The embodiments and modifications of the present invention are specifically described above. However, the present invention is not limited to the above-described embodiments and modifications and can be changed without departing from the gist of the present invention.

### List of Reference Signs

1A: immunoanalyzer (automatic analyzer)
10: flow channel
11, 11a, 11b, 12: channel wall
13: magnetic particle
14: capture region
15: reaction field electrode
16: counter electrode
17a, 17b: lead wire
18: voltage application means
19: magnet
20, 20A, 20B, 20C: slide mechanism
21: photodetector
22: condenser lens
23: laser light source
24, 24A, 24B, 24C: magnet guide mechanism
25: stopper structure
26: movable structure
26a: shaft
26b: guide member
28: outer shell
29: deformable portion
30, 31, 32: tube
33: sipper nozzle
34: arm
35: pump (supply means, discharge means)
36, 37: valve
40: suspension container
41: reaction unit
42: cleaning liquid container
43: buffer solution container
44: cleaning mechanism
45: waste liquid container
50: controller
51,52,53,54,55,56,57,58: signal line
61: projection plane (projection plane on which the capture region is projected)
O: center (center of magnet)
P: capture operation position

## Claims

1. An automatic analyzer comprising:
a flow channel through which a sample containing a magnetic particle bound to a specific substance is introduced into a capture region;
supply means for supplying the sample to the flow channel;
a magnet that captures the magnetic particle;
a slide mechanism that moves the magnet to a vicinity of a projection plane on which the capture region is projected;
a detector that detects the specific substance on the magnetic particle;
discharge means for discharging the magnetic particle from the flow channel after the detection by the detector; and
a magnet guide mechanism that is formed from a magnetic material and moves the magnet.

2. The automatic analyzer according to claim 1, wherein
the magnetic material is a soft magnetic material.

3. The automatic analyzer according to claim 1, wherein
at least a portion of the slide mechanism is formed from an elastic material.

4. The automatic analyzer according to claim 1, wherein
the magnet guide mechanism is located below a center of the magnet when the magnet is at a capture operation position.

5. The automatic analyzer according to claim 1, further comprising
a stopper structure that stops the magnet moved by the magnet guide mechanism.

6. The automatic analyzer according to claim 5, wherein
the stopper structure has a shape that simultaneously contacts the magnet in at least two or more places.

7. The automatic analyzer according to claim 1, wherein
the magnet guide mechanism simultaneously contacts the magnet in at least two or more places.

8. The automatic analyzer according to claim 7, wherein
the magnet guide mechanism is fixed in a wall of the flow channel by insert molding.

9. The automatic analyzer according to claim 1, wherein
the magnet guide mechanism shares a shape with at least one or more edges of the magnet, in the projection plane in at least one or more directions.

10. The automatic analyzer according to claim 9, wherein
the magnet guide mechanism is a member fixed near the flow channel, and there are at least two or more magnet guide mechanisms.

11. The automatic analyzer according to claim 1, wherein
the slide mechanism has a movable structure in part, the movable structure allowing the slide mechanism to move in at least one or more axial directions.

12. The automatic analyzer according to claim 1, wherein
the slide mechanism includes an outer shell that at least partially encapsulates the magnet, and
a gap is formed between the magnet and the outer shell, and the magnet moves within the outer shell.

13. An automatic analyzer comprising:
a flow channel through which a sample containing a magnetic particle bound to a specific substance is introduced into a capture region;
supply means for supplying the sample to the flow channel;
a magnet that captures the magnetic particle; a slide mechanism that moves the magnet to a vicinity of a projection plane on which the capture region is projected;
a detector that detects the specific substance on the magnetic particle; and
discharge means for discharging the magnetic particle from the flow channel after the detection by the detector, wherein
the magnet moved by driving the slide mechanism is stopped by being pushed against a magnet guide mechanism that is located near the projection plane on which the capture region is projected.

## Amended claims

### Amended claims under Art. 19.1 PCT

1. An automatic analyzer comprising:
a flow channel through which a sample containing a magnetic particle bound to a specific substance is introduced into a capture region;
supply means for supplying the sample to the flow channel;
a magnet that captures the magnetic particle;
a slide mechanism that moves the magnet to a vicinity of a projection plane on which the capture region is projected;
a detector that detects the specific substance on the magnetic particle;
discharge means for discharging the magnetic particle from the flow channel after the detection by the detector; and
a magnet guide mechanism that is formed from a magnetic material and moves the magnet.

2. The automatic analyzer according to claim 1, wherein
the magnetic material is a soft magnetic material.

3. The automatic analyzer according to claim 1, wherein
at least a portion of the slide mechanism is formed from an elastic material.

4. The automatic analyzer according to claim 1, wherein
the magnet guide mechanism is located below a center of the magnet when the magnet is at a capture operation position.

5. The automatic analyzer according to claim 1, further comprising
a stopper structure that stops the magnet moved by the magnet guide mechanism.

6. The automatic analyzer according to claim 5, wherein
the stopper structure has a shape that simultaneously contacts the magnet in at least two or more places.

7. The automatic analyzer according to claim 1, wherein
the magnet guide mechanism simultaneously contacts the magnet in at least two or more places.

8. The automatic analyzer according to claim 7, wherein
the magnet guide mechanism is fixed in a wall of the flow channel by insert molding.

9. The automatic analyzer according to claim 1, wherein
the magnet guide mechanism shares a shape with at least one or more edges of the magnet, in the projection plane in at least one or more directions.

10. The automatic analyzer according to claim 9, wherein
the magnet guide mechanism is a member fixed near the flow channel, and there are at least two or more magnet guide mechanisms.

11. The automatic analyzer according to claim 1, wherein
the slide mechanism has a movable structure in part, the movable structure allowing the slide mechanism to move in at least one or more axial directions.

12. The automatic analyzer according to claim 1, wherein
the slide mechanism includes an outer shell that at least partially encapsulates the magnet, and
a gap is formed between the magnet and the outer shell, and the magnet moves within the outer shell.

Statement under Art. 19.1 PCT
Claim 13 originally filed has been deleted.
